# EUROPEAN PATENT APPLICATION

(11) **EP 1 489 186 A1**
(43) Date of publication of application: **22.12.2004**
(21) Application number: 03710259.7
(22) Date of filing: 06.03.2003
(51) Int. Cl.: C12P 17/02, C07D 493/04, C07D 303/32, A61P 17/06, A61P 27/02, A61P 29/00, A61P 35/00, A61P 43/00

(54) **ANGIOGENESIS INHIBITORS**

(30) Priority: 08.03.2002 JP 2002063059
(71) Applicant: MERCIAN CORPORATION, Chuo-ku, Tokyo 104-8305 (JP); ZAIDAN HOJIN BISEIBUTSU KAGAKU KENKYU KAI, Shinagawa-ku, Tokyo 141-0021 (JP)
(72) Inventor: KUMAGAI, Hiroyuki, Chigasaki-shi, Kanagawa 253-0035 (JP); SAMESHIMA, Tomohiro, Fujisawa-shi, Kanagawa 251-0052 (JP); MATSUFUJI, Motoko, Komae-shi, Tokyo 201-0005 (JP); KAWAMURA, Naoto, Yamato-shi, Kanagawa 242-0007 (JP); SOMENO, Tetsuya, Saitama-shi, Saitama 330-0825 (JP); ISHIZUKA, Masaaki, Ohta-ku, Tokyo 145-0072 (JP); TAKEUCHI, Tomio, Minato-ku, Tokyo 108-0073 (JP)
(74) Representative: Horner, Martin Grenville
(86) International application number: PCT/JP2003/002634
(87) International publication number: WO 2003/076638

(57) **Abstract**

Compounds represented by formula (I) below; a process for producing the compounds by culturing a microorganism belonging to the genus Aspergillus and isolating the above-mentioned compounds from the culture; an angiogenesis inhibitory agent containing as an active ingredient the compounds; and an Aspergillus sp. F-1491 (FERM BP-8288) strain capable of producing the compounds. 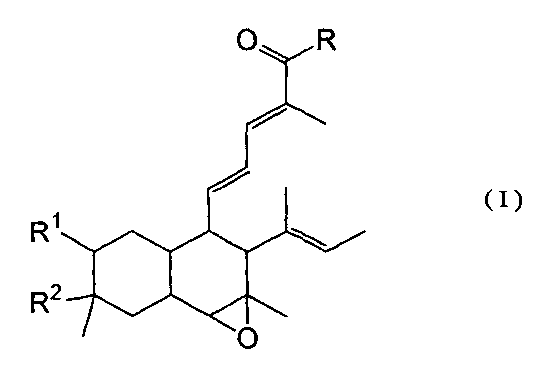 In formula (I), R represents a methyl group or an ethyl group, R¹ represents a hydrogen atom, a chlorine atom, a hydroxyl group or a methoxy group, R² represents a hydroxyl group, or R¹ and R² taken together form an epoxy ring structure.

## Description

### TECHNICAL FIELD

The present invention relates to novel compounds produced by a microorganism belonging to the genus *Aspergillus* having an angiogenesis inhibiting activity, a process for producing the compounds by cultivating a microorganism having the capability of producing the compounds and isolating the compounds from the culture broth, angiogenesis inhibitory agents containing the compounds as an active ingredient, and *Aspergillus* sp. F-1491 (FERM BP-8288) strain having the capability of producing the compounds.

### BACKGROUND ART

Angiogenesis generally means a phenomenon that involves: digestion or destruction of a basal lamina of a blood vessel by protease; migration, proliferation, and adhesion to an extracellular matrix of angioendothelial cells; lumen genesis by differentiation of angioendothelial cells; and reconstitution of blood vessels. In a physiological aspect, angiogenesis occurs upon luteinization and placentation. Angiogenesis also occurs in diseases such as solid cancers, diabetic retinopathy and chronic inflammatory diseases. For example, in retinopathy, angiogenesis proceeds as follows. That is, the retinal tissue interposed between a basal lamina around existing retinal blood vessels and the vitreous body are first destructed. Then the angioendothelial cells that constitute the existing blood vessels migrate through the interstices at the destructed portion of the retinal tissue. Angioendothelial cells proliferate to fill the vacancy resulted from the migration of the angioendothelial cells. Then the angioendothelial cells that have migrated to the vitreous body of retina reconstitute blood vessels. Further, it is essential for proliferation of a solid cancer to secure a route by angiogenesis, through which supply of nutrition and oxygen and removal of wastes are performed. Furthermore, in metastasis, which is a large problem in the current therapy of cancers, angiogenesis is an important step in the sense that angiogenesis secures that route.

Since angiogenesis is involved deeply in the onset and progress of various diseases as described above, studies for discovering substances that inhibit angiogenesis have been vigorously promoted for the purpose of prevention and treatment for such diseases. Examples of known angiogenesis inhibitors include drugs such as fumagillin analogues, which are metabolites of microorganisms that have an effect of inhibiting the proliferation of angioendothelial cells; tetracycline antibiotics that have an effect of inhibiting the collagenase activity; and microorganism-derived D-glucogalactan sulfate that has an inhibitory action on binding of heparin-binding angiogenesis factors to receptors. Some of those substances are being studied on their clinical effectiveness.

However, at present, there are no satisfying therapeutic methods for the above-mentioned diseases involved in angiogenesis since there are no drugs that are clinically effective as angiogenesis inhibitory agents. In particular, in diabetic retinopathy, development of drugs that exhibit excellent effects against angiogenesis has been keenly desired, because no regression of newly formed blood vessels is attained unless surgical treatment is performed and sight disorder due to bleeding from the newly formed blood vessels is a matter of concern.

### DISCLOSURE OF THE INVENTION

It is an object of the present invention to provide a novel compound that have an angiogenesis inhibiting activity, a process for producing such a compound, a novel microorganism that is capable of producing such a compound, and angiogenesis inhibitory agents that contain such a compound as an active ingredient.

To attain the above-mentioned object, the present inventors have isolated microorganisms from soil of various geographical areas and have extensively studied metabolites produced by the microorganisms. As a result, they have found that a newly isolated microorganism belonging to the genus *Aspergillus* produces in the culture broth a substance that shows an angiogenesis inhibiting activity. Separation and purification of the active substance from the culture broth and investigation of the physicochemical properties of the active substance confirmed that the obtained active substance differs from any known substances and has an excellent angiogenesis inhibiting activity. Thus, the present invention has been completed.

That is, the present invention relates to: novel compounds having an angiogenesis inhibiting activity represented by formula (I) below; a process for producing the compounds by culturing a microorganism belonging to the genus *Aspergillus* and isolating the above-mentioned compounds from the culture broth; and Aspergillus sp. F-1491 (FERM BP-8288) strain that has the capability of producing the compounds.
1. Compounds represented by formula (I) 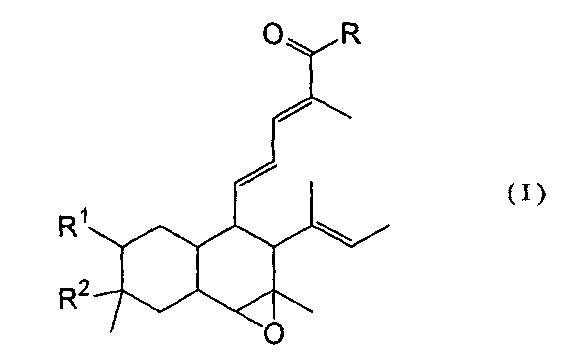 wherein R represents a methyl group or an ethyl group, R¹ represents a hydrogen atom, a chlorine atom, a hydroxyl group or a methoxy group, R² represents a hydroxyl group, or R¹ and R² taken together form an epoxy ring structure.
2. The compound according to 1 above, in which in formula (I), R represents an ethyl group, and R¹ and R² taken together form an epoxy ring structure.
3. The compound according to 1 above, in which in formula (I), R represents a methyl group, and R¹ and R² taken together form an epoxy ring structure.
4. The compound according to 1 above, in which in formula (I), R represents an ethyl group, R¹ represents a chlorine atom, and R² represents a hydroxyl group.
5. The compound according to 1 above, in which in formula (I), R represents a methyl group, R¹ represents a chlorine atom, and R² represents a hydroxyl group.
6. The compound according to 1 above, in which in formula (I), R represents an ethyl group, R¹ represents a hydrogen atom, and R² represents a hydroxyl group.
7. The compound according to 1 above, in which in formula (I), R represents a methyl group, R¹ represents a hydrogen atom, and R² represents a hydroxyl group.
8. The compound according to 1 above, in which in formula (I), R represents a methyl group, R¹ represents a methoxy group, and R² represents a hydroxyl group.
9. The compound according to 1 above, in which in formula (I), R represents a methyl group, and R¹ and R² represent a hydroxyl group each.
10. A process for producing the compounds according to 1 above, characterized by:
   culturing a microorganism having the capability of producing the compounds according to 1 above; and
   isolating the compounds according to 1 above from the culture broth of the microorganism.
11. An Aspergillus sp. F-1491 (FERM BP-8288) strain having the capability of producing the compounds according to 1 above.
12. An angiogenesis inhibitory agent containing the compounds according to 1 above as an active ingredient.

### BRIEF DESCRIPTION OF THE DRAWINGS

Fig. 1 is a diagram showing an infrared absorption spectrum of the substance F-1491A by the KBr method.
Fig. 2 is a diagram showing an infrared absorption spectrum of the substance F-1491B by the KBr method.

### DETAILED DESCRIPTION

The inventors of the present invention have named specific examples of the compounds represented by formula (I) as follows.
(1) A compound in which R represents an ethyl group, and R¹ and R² taken together form an epoxy ring structure: F-1491A,
(2) A compound in which R represents a methyl group, and R¹ and R² taken together form an epoxy ring structure: F-1491B,
(3) A compound Compound in which R represents an ethyl group, R¹ represents a chlorine atom, and R² represents a hydroxyl group: F-1491C,
(4) A compound in which R represents a methyl group, R¹ represents a chlorine atom, and R² represents a hydroxyl group: F-1491D,
(5) A compound in which R represents an ethyl group, R¹ represents a hydrogen atom, and R² represents a hydroxyl group: F-1491E,
(6) A compound in which R represents a methyl group, R¹ represents a hydrogen atom, and R² represents a hydroxyl group: F-1491F,
(7) A compound in which R represents a methyl group, R¹ represents a methoxy group, and R² represents a hydroxyl group: F-1491G,
(8) A compound in which R represents a methyl group, and R¹ and R² represent a hydroxyl group each: F-1491H.

Hereinafter, in the present specification, explanation of those compounds will be made using the above-mentioned names or those substances will be collectively referred to as the substance F-1491.

The substances F-1491 have structural similarity to Fusarielins that have been reported to have a weak antifungal activity (J. Antibiotics, 48(1), 45-52 (1995)). However, the substances F-1491 of the present invention are novel compounds that are clearly distinguished in the molecular formulae, physicochemical properties, structures and biological effects thereof from the known Fusarielins.

Further, the present invention provides: a process for producing the compound by culturing a microorganism having the capability of producing the compound represented by formula (I) having an angiogenesis inhibiting activity and isolating the compound from the culture broth; a microorganism having the capability of producing the compound and belonging to the genus *Aspergillus*; and angiogenesis inhibitory agents containing the compound as an active ingredient.

The microorganism that is used in the present invention may be any strain that belongs to the genus Aspergillus, and that has the capability of producing the substances F-1491 of the present invention. Search for such a microorganism may be performed, for example, as follows. That is, each extract from culture broths of various microorganisms is added onto a microplate on which angioendothelial cells proliferate and uptake of radioisotope-labeled thymidine or the like into the cells, which is an index of proliferation, is measured. Isolating and identifying an active substance from the culture broth of the microorganism in which a decrease in the amount of the uptake is observed, that is, the proliferation of angioendothelial cells is inhibited can provide a microorganism that has the capability of producing the target substance F-1491.

Examples of the microorganism found in this manner include a strain F-1491 that was isolated from soil by the present inventors and that belongs to the genus *Aspergillus*. However, the microorganism is not limited to this strain. All the strains can be used in the present invention so far as they belong to the genus *Aspergillus* and have the capability of producing the substance F-1491 of the present invention, including mutants of the strains, for example, artificial mutants obtained by treatments with mutagens such as ultraviolet rays, X-rays, radiations and chemicals as well as spontaneous mutants.

Hereinafter, taxonomical properties of the strain F-1491 will be explained.

Inoculation of this strain on various plate media to say, Czapek yeast extract agar (hereinafter, referred to as "CYA"), malt extract agar (hereinafter, referred to as "MEA"), and Czapek yeast extract agar containing 20% sucrose (hereinafter, referred to as "CY20S") and incubation at 25°C for seven days result in flat to slightly uprising growth on all the agar plates. The hyphae were thick floccus-like to felt-like, the color of which is white to reddish gray (8A-B1-2). The rear side has substantially the same color tone as that of the front side. Change of the color tone of the front side by formation of conidia presents grayish green (25C-D5-6) in MEA and CY20S plates. The growth rate is different on different media; under the cultivation conditions of 25°C for one week, the strain reaches a diameter of 56 to 58 mm on the CYA plate, a diameter of 24 to 26 mm on the MEA plate, and a diameter of 28 to 29 mm on the CY20S plate. On the CYA plate, growth of 12 to 14 mm is observed at 37°C. On the CYA plate after prolonged cultivation, some amount of transparent exudate is observed. No production of soluble pigments is observed on all the plates. Note that the descriptions on color tones are made according to Methuen Handbook of Colour (Kornerup & Wanscher, 1978).

Observation of the morphological characteristics of the strain under an optical microscope showed their conidial structure that was uniseriate and similar to that of Aspergillus. The strigmata are of Phialo type and ampoule-shaped with short necks. The stipe has no septum and is colorless, smooth and extremely short. A number of stipes of 40 to 80 µm × 2.5 to 3.0 µm are observed. In the connecting portions between the stipes and vegetative mycelium, existence of foot cells is observed. At the apices of the stipes, vesicles arise, which are subglobose to flask-shaped. Most of the vesicles have a width of 10 to 15 µm. No metula is observed. The phialide arises at one half from the apex of a vesicle, grows upwards, and is ampoule-shaped and smooth. The size of the phialide is 6.0 to 7.5 µm × 1.8 to 2.5 µm. The conidia are unicellular and most of them are globose. The conidia have a smooth surface and a size of 2.5 to 3.5 µm. The conidia line up together in the form of a chain at the time of formation but no clear connecting portion is observed. No formation of sexual reproduction organ such as cleistothecium is observed from samples after four weeks from the start of the culture.

From the taxonomical properties described above, the present inventors have judged that the strain belongs to the genus *Aspergillus*, named it *Aspergillus* sp. F-1491, and deposited it on October 2, 2001 at the independent administrative corporation, National Institute of Advanced Industrial Science and Technology, International Patent Organism Depositary at Chuo Dai-6, 1-1 Higashi 1-Chome, Tsukuba-shi, Ibaraki-ken, Japan, as the accession number of FERM P-18549, and transferred from the original deposit to international deposit based on Budapest Treaty on January 31, 2003, and deposited as the accession number of FERM BP-8288.

The substances F-1491 of the present invention can be produced by inoculating the above-mentioned strain in a nutrient source-containing medium and culturing it aerobically. The substance F-1491-producing microorganism is not limited to the above-mentioned strain but all the strains that belong to the genus *Aspergillus* and have the capability of producing the substances F-1491 may be used in the present invention.

The method of cultivating the above-mentioned microorganisms is in principle pursuant to the cultivation method for general microorganisms. Usually, it is preferable that the method is practiced under aerobic conditions, such as shaking culture with liquid medium or aeration agitation culture. The medium that is used for cultivation may be any medium that contains nutrient source available to the microorganisms belonging to the genus *Aspergillus*. Various synthetic and semi-synthetic media as well as natural media are available. The composition of medium is as follows. Carbon sources including glucose, sucrose, fructose, glycerol, dextrin, starch, molasses and so on may be used singly or in combination. Nitrogen sources including organic nitrogen sources such as pharmamedia, peptone, meat extract, soybean powder, casein, amino acids, yeast extract and urea may be used singly or in combination. In addition, salts such as sodium chloride, potassium chloride, calcium carbonate, magnesium sulfate, sodium phosphate, potassium phosphate and cobalt chloride, heavy metal salts, vitamins such as vitamin B and biotin may be added if necessary.

In the case where considerable foaming occurs during the cultivation, various defoaming agents may be added in the medium appropriately. When the defoaming agent is added, care must be taken to add the defoaming agent in a concentration that does not adversely affect production of the target substance. It is desirable that the pH of the medium is about 5 to about 9, usually around neutrality. It is recommended to keep the cultivation temperature at usually 10 to 40°C, preferably 20 to 27°C. The cultivation time is about 2 to 14 days, usually 3 to 5 days. Needless to say, various cultivation conditions described above may be varied depending on the kind and characteristics of the microorganism used, external conditions, and so on as appropriate and optimal conditions can be selected. The substances F-1491 of the present invention which accumulates in the culture broth can be isolated by separating the mycelium by using known ordinary solid-liquid separation means such as filtration and centrifugation; or extracting the target compound from each of the supernatant and the mycelium.

Separation and purification of the substance F-1491 can be performed by selecting and combining various known methods. For example, a solvent extraction method using ethyl acetate, methanol, n-butanol or the like, and a column chromatographic method using a solid phase, for example, polystyrene-based adsorbent resin such as Amberlite XAD (manufactured by Roam and Haas Co., Ltd.) or Diaion HP-20 (manufactured by Mitsubishi Chemical Corporation), silica gel, alumina, or activated carbon may be used. The method of eluting the target substance from a solid phase may vary depending on the kind and properties of a solid phase. For example, in the case of polystyrene-based adsorbent resin, hydroalcohol, hydroacetone and the like can be used as eluting solvents. Further, gel filtration using Sephadex LH-20 (manufactured by Pharmacia AB), Bio Gel P-2 (manufactured by Bio-Rad Laboratories) or the like, thin layer chromatography using silica gel, alumina or the like, preparative high performance liquid chromatography (preparative HPLC) using a normal phase or reversed phase column and so on may be used. These methods may be used singly or in combination as appropriate, or in repetitions as appropriate to effect separation and purification.

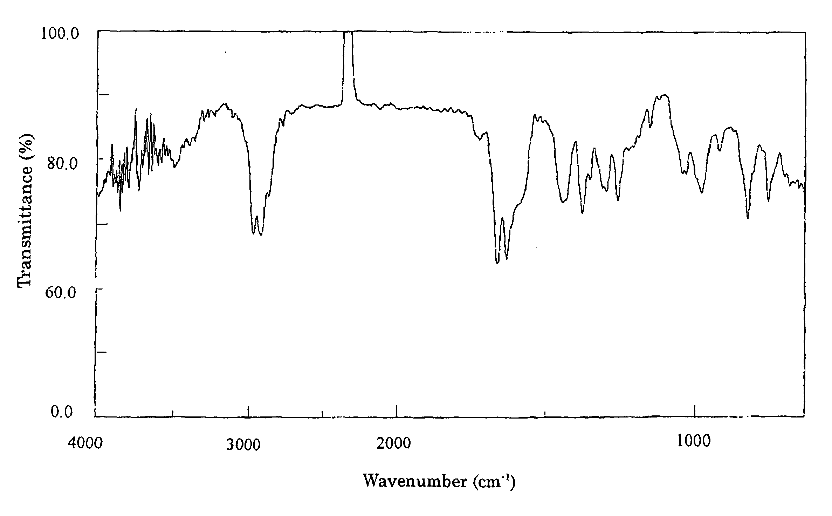

The substances F-1491A to F-1491H thus obtained have the following physicochemical properties.
1. Physicochemical properties of the substance F-1491A
(1) Form: white powder
(2) Molecular formula: C₂₄H₃₄O₃
   (Measurement of C₂₄H₃₅O₃ by high resolution FAB mass spectrometry Calculated m/z: 371.2586 (M+H)⁺, Found m/z: 371.2580)
(3) Specific optical rotation: [α]_{D}²⁶ -194° (c 0.1, methanol)
(4) Melting point: 55 to 58°C
(5) Infrared absorption spectrum: The results obtained by measurement by the KBr method are as shown in Fig. 1. The characteristic absorptions are as follows.
   IRνₘₐₓ(KBr) cm⁻¹: 2970, 2915, 1660, 1630, 1440, 1375, 1260, 835
(6) Ultraviolet absorption spectrum (measured in methanol):
   UVλₘₐₓ nm: 280
(7) Solubility: Soluble in methanol, chloroform and dimethyl sulfoxide; hardly soluble in water
(8) ¹H-NMR spectrum: Measurement was preformed by dissolving the substance in deuterated methanol and using tetramethylsilane as an internal standard. The chemical shift, multiplicity and spin coupling constant of each signal are shown in Table 1.
(9) ¹³C-NMR spectrum: Measurement was performed by dissolving the substance in deuterated methanol and using tetramethylsilane as an internal standard. The chemical shift and multiplicity of each signal are shown in Table 2.

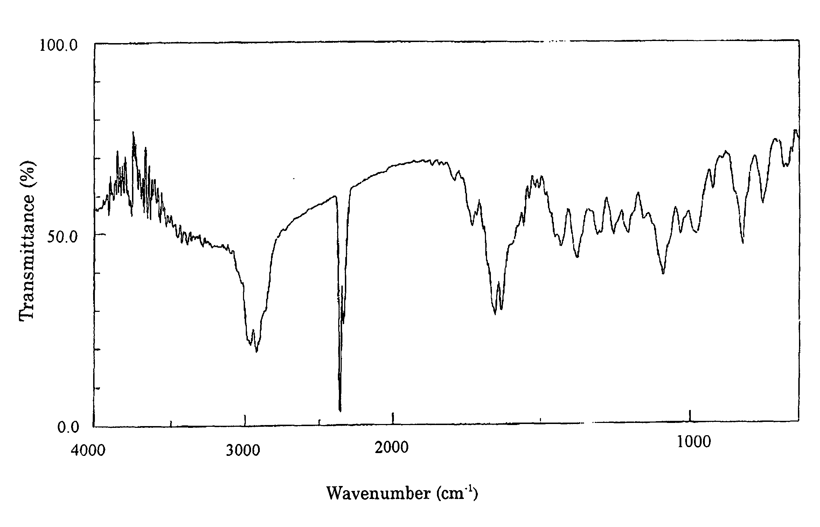

2. Physicochemical properties of the substance F-1491B
(1) Form: white powder
(2) Molecular formula: C₂₃H₃₂O₃
   (Measurement of C₂₃H₃₃O₃ by high resolution FAB mass spectrometry Calculated m/z: 357.2430 (M+H)⁺, Found m/z: 357.2463),
(3) Specific optical rotation: [α]_{D}²⁶ -222° (c 0.1, methanol)
(4) Melting point: 53 to 56°C
(5) Infrared absorption spectrum: The results obtained by measurement by the KBr method are as shown in Fig. 2. The characteristic absorptions are as follows.
   IRνₘₐₓ(KBr) cm⁻¹: 2965, 2925, 1655, 1630, 1435, 1375, 1255, 835
(6) Ultraviolet absorption spectrum (measured in methanol):
   UVλₘₐₓ nm: 280
(7) Solubility: Soluble in methanol, chloroform and dimethyl sulfoxide; hardly soluble in water
(8) ¹H-NMR spectrum: Measurement was performed by dissolving the substance in deuterated methanol and using tetramethylsilane as an internal standard. The chemical shift, multiplicity, and spin coupling constant of each signal are shown in Table 1.
(9) ¹³C-NMR spectrum: Measurement was performed by dissolving the substance in deuterated methanol and using tetramethylsilane as an internal standard. The chemical shift and multiplicity of each signal are shown in Table 2.

3. Physicochemical properties of the substance F-1491C
(1) Form: white powder
(2) Molecular formula: C₂₄H₃₅O₃Cl
   (Measurement of C₂₄H₃₅O₃Cl by high resolution ESI mass spectrometry Calculated m/z: 429.2172 (M+Na)⁺, Found m/z: 429.2204)
(3) Ultraviolet absorption spectrum (measured in methanol):
   UVλₘₐₓ nm: 280
(4) ¹H-NMR spectrum: Measurement was performed by dissolving the substance in deuterated methanol and using tetramethylsilane as an internal standard. The chemical shift, multiplicity and spin coupling constant of each signal are shown in Table 1.
(5) ¹³C-NMR spectrum: Measurement was performed by dissolving the substance in deuterated methanol and using tetramethylsilane as an internal standard. The chemical shift and multiplicity of each signal are shown in Table 2.

4. Physicochemical properties of the substance F-1491D
(1) Form: white powder
(2) Molecular formula: C₂₃H₃₃O₃Cl
   (Measurement of C₂₃H₃₃O₃Cl by high resolution ESI mass spectrometry Calculated m/z: 415.2016 (M+Na)⁺, Found m/z: 415.2039)
(3) Ultraviolet absorption spectrum (measured in methanol):
   UVλₘₐₓ nm: 280
(4) ¹H-NMR spectrum: Measurement was performed by dissolving the substance in deuterated chloroform and using tetramethylsilane as an internal standard. The chemical shift, multiplicity and spin coupling constant of each signal are shown in Table 1.
(5) ¹³C-NMR spectrum: Measurement was performed by dissolving the substance in deuterated chloroform and using tetramethylsilane as an internal standard. The chemical shift and multiplicity of each signal are shown in Table 2.

5. Physicochemical properties of the substance F-1491E
(1) Form: white powder
(2) Molecular formula: C₂₄H₃₆O₃
   (Measurement of C₂₉H₃₆O₃ by high resolution ESI mass spectrometry Calculated m/z: 395.2562 (M+Na)⁺, Found m/z: 395.2560)
(3) Ultraviolet absorption spectrum (measured in methanol):
   UVλₘₐₓ nm: 280
(4) ¹H-NMR spectrum: Measurement was performed by dissolving the substance in deuterated methanol and using tetramethylsilane as an internal standard. The chemical shift, multiplicity and spin coupling constant of each signal are shown in Table 1.
(5) ¹³C-NMR spectrum: Measurement was performed by dissolving the substance in deuterated methanol and using tetramethylsilane as an internal standard. The chemical shift and multiplicity of each signal are shown in Table 2.

6. Physicochemical properties of the substance F-1491F
(1) Form: white powder
(2) Molecular formula: C₂₃H₃₄O₃
   (Measurement of C₂₃H₃₄NO₃ by high resolution ESI mass spectrometry Calculated m/z: 381.2406 (M+Na)⁺, Found m/z: 381.2408)
(3) Ultraviolet absorption spectrum (measured in methanol):
   UVλₘₐₓ nm: 280
(4) ¹H-NMR spectrum: Measurement was performed by dissolving the substance in deuterated methanol and using tetramethylsilane as an internal standard. The chemical shift, multiplicity and spin coupling constant of each signal are shown in Table 1.
(5) ¹³C-NMR spectrum: Measurement was performed by dissolving the substance in deuterated methanol and using tetramethylsilane as an internal standard. The chemical shift and multiplicity of each signal are shown in Table 2.

7. Physicochemical properties of the substance F-1491G
(1) Form: white powder
(2) Molecular formula: C₂₄H₃₆O₄
   (Measurement of C₂₄H₃₆O₄ by high resolution ESI mass spectrometry Calculated m/z: 411.2511 (M+Na)⁺, Found m/z: 411.2533)
(3) Ultraviolet absorption spectrum (measured in methanol):
   UVλₘₐₓ nm: 280
(4) ¹H-NMR spectrum: Measurement was performed by dissolving the substance in deuterated methanol and using tetramethylsilane as an internal standard. The chemical shift, multiplicity and spin coupling constant of each signal are shown in Table 1.
(5) ¹³C-NMR spectrum: Measurement was performed by dissolving the substance in deuterated methanol and using tetramethylsilane as an internal standard. The chemical shift and multiplicity of each signal are shown in Table 2.

8. Physicochemical properties of the substance F-1491H
(1) Form: white powder
(2) Molecular formula: C₂₃H₃₄O₄
   (Measurement of C₂₃H₃₄O₄ by high resolution ESI mass spectrometry Calculated m/z: 397.2355 (M+Na)⁺, Found m/z: 397.2376)
(3) Ultraviolet absorption spectrum (measured in methanol):
   UVλₘₐₓ nm: 280
(4) ¹H-NMR spectrum: Measurement was performed by dissolving the substance in deuterated chloroform and using tetramethylsilane as an internal standard. The chemical shift, multiplicity and spin coupling constant of each signal are shown in Table 1.
(5) ¹³C-NMR spectrum: Measurement was performed by dissolving the substance in deuterated chloroform and using tetramethylsilane as an internal standard. The chemical shift and multiplicity of each signal are shown in Table 2.

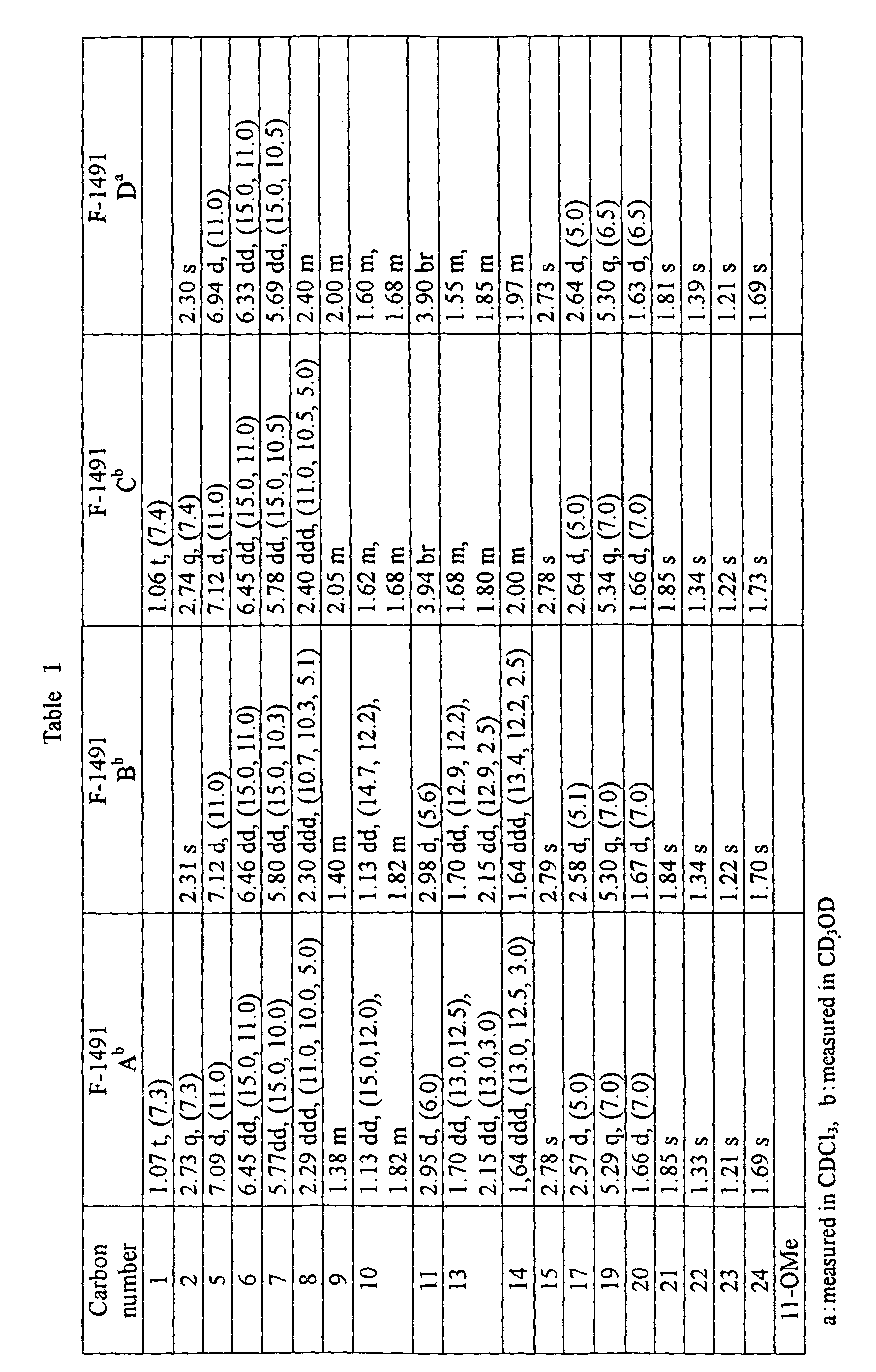

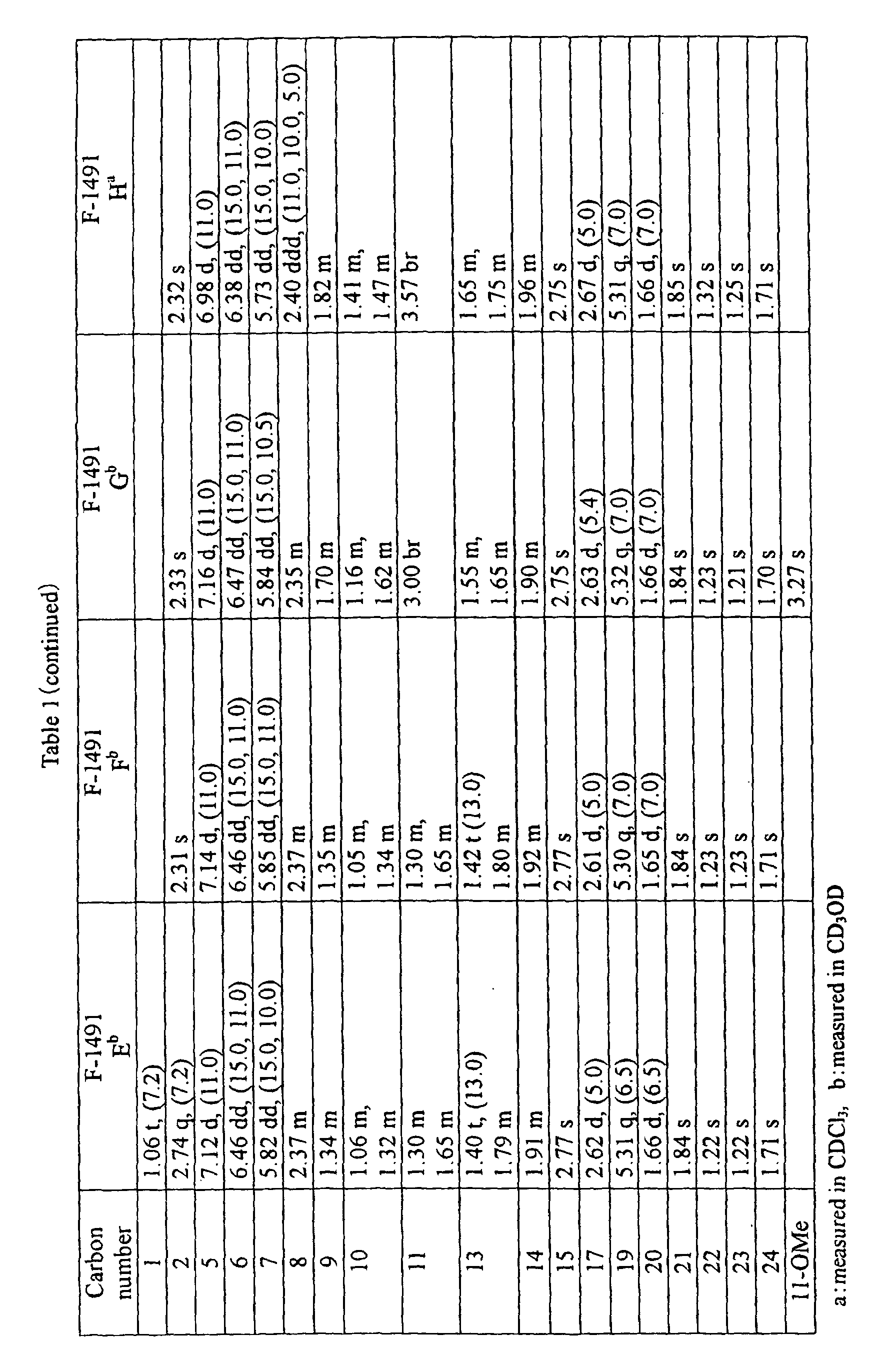

**Table 2**

| Carbon number | F-1491 A^{b} | F-1491 B^{b} | F-1491 C^{b} | F-1491 D^{a} | F-1491 E^{b} | F-1491 F^{b} | F-1491 G^{b} | F-1491 H^{a} |
|---|---|---|---|---|---|---|---|---|
| 1 | 9.2 q | - | 9.3 q | - | 9.3 q | - | - | - |
| 2 | 30.8 t | 25.6 q | 31.3 t | 25.6 q | 31.3 t | 25.6 q | 25.6 q | 25.6 q |
| 3 | 205.2 s | 202.4 s | 205.1 s | 200.0 s | 205.1 s | 202.5 s | 202.5 s | 199.9 s |
| 4 | 135.3 s | 136.0 s | 135.2 s | 135.1 s | 134.9 s | 135.6 s | 135.7 s | 135.0 s |
| 5 | 139.9 d | 141.1 d | 139.9 d | 139.0 d | 140.1 d | 141.6 s | 141.5 d | 139.1 d |
| 6 | 128.6 d | 128.6 d | 128.7 d | 127.5 d | 128.3 d | 128.3 d | 128.6 d | 127.3 d |
| 7 | 146.5 d | 146.7 d | 146.6 d | 144.9 d | 147.6 d | 148.0 d | 147.6 d | 145.6 d |
| 8 | 45.4 d | 45.4 d | 44.7 d | 43.7 d | 46.1 d | 46.1 d | 45.7 d | 44.2 d |
| 9 | 34.3 d | 34.1 d | 31.5 d | 30.2 d | 38.5 d | 38.5 d | 31.3 d | 30.1 d |
| 10 | 31.6 t | 31.6 t | 36.0 t | 34.9 t | 28.0 t | 28.0 t | 29.8 t | 34.4 t |
| 11 | 61.3d | 61.4d | 66.7 d | 65.3 d | 40.0 t | 40.0 t | 84.4 d | 74.0 d |
| 12 | 60.2 s | 60.1 s | 73.2 s | 72.9 s | 70.7 s | 70.7 s | 72.7 s | 72.2 s |
| 13 | 36.8 t | 36.9 t | 39.1 t | 38.2 t | 44.8 t | 44.8 t | 40.5 t | 39.2 t |
| 14 | 35.2 d | 35.3 d | 37.9 d | 36.7 d | 38.5 d | 38.5 d | 38.1 d | 37.0 d |
| 15 | 64.5 d | 64.5 d | 65.5 d | 63.9 d | 66.0 d | 66.0 d | 65.9 d | 64.1 d |
| 16 | 62.5 s | 62.5 s | 62.6 s | 61.3 s | 62.7 s | 62.7 s | 62.6 s | 61.5 s |
| 17 | 54.8 d | 54.6 d | 55.1 d | 53.5 d | 55.0 d | 55.0 d | 55.2 d | 53.1 d |
| 18 | 134.2 s | 134.1 s | 134.2 s | 132.5 s | 134.6 s | 134.5 s | 134.4 s | 132.8 s |
| 19 | 126.9 d | 127.0 d | 127.3 d | 126.2 d | 128.0 d | 126.9 d | 127.1 d | 125.6 d |
| 20 | 13.6 q | 13.6 q | 13.6 q | 13.6 q | 13.6 q | 13.6 q | 13.6 q | 13.7 q |
| 21 | 11.7 q | 11.5 q | 11.7 q | 11.4 q | 11.7 q | 11.4q | 11.4q | 11.5 q |
| 22 | 23.1 q | 23.0 q | 29.0 q | 29.2 q | 31.6q | 31.6q | 27.7 q | 27.7 q |
| 23 | 22.1 q | 22.1 q | 22.4 q | 22.0 q | 22.4 q | 22.4 q | 22.4 q | 22.0 q |
| 24 | 19.5 q | 19.5 q | 18.9 q | 19.0 q | 19.0 q | 19.0 q | 19.5 q | 19.5 q |
| 11-OMe | - | - | - | - | - | - | 57.2 q | - |
| a: measured in CDCl₃, b: measured in CD₃OD | | | | | | | | |

The results of analysis of various spectra led to determination of the structures of the substances F-1491A, F-1491B, F-1491C, F-1491D, F-1491E, F-1491F, F-1491G and F-1491H as shown below.

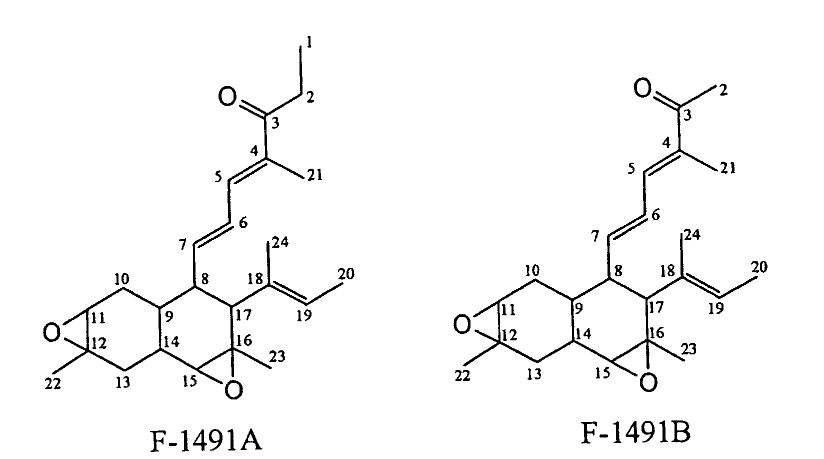

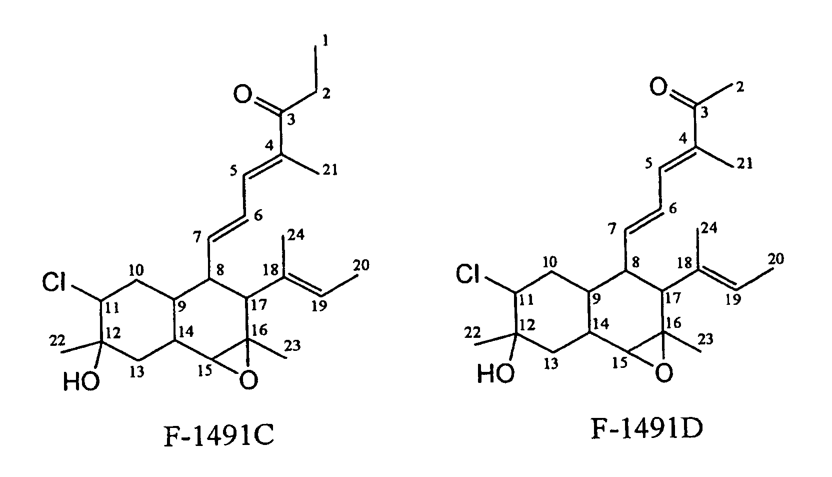

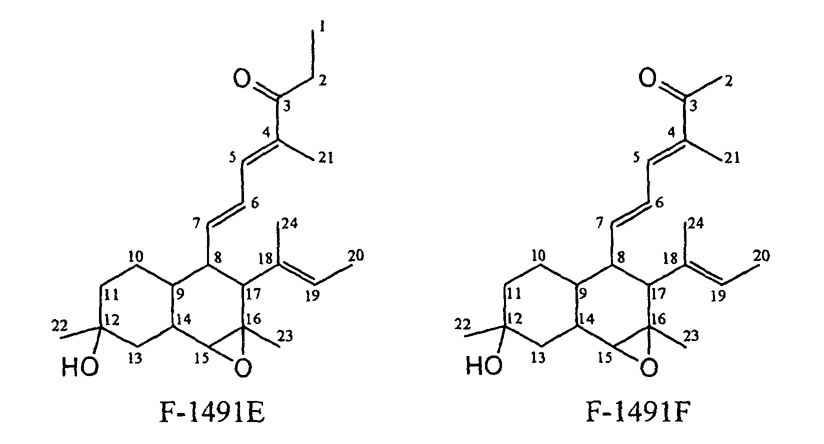

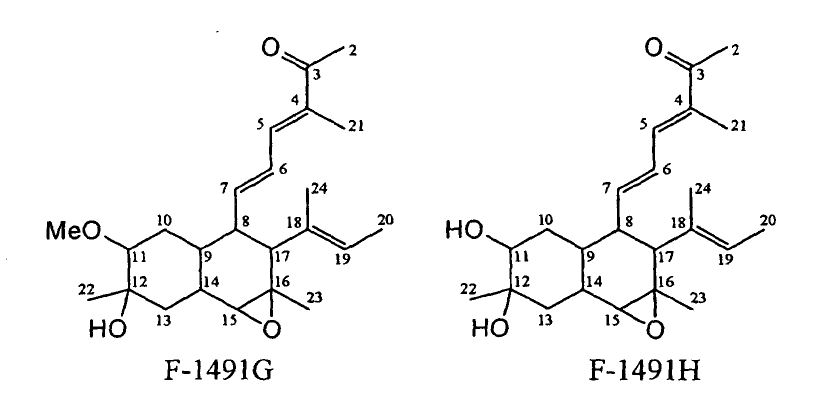

The series of substances F-1491 (substances F-1491A to F-1491H) has excellent angiogenesis inhibiting activity and in addition low toxicity to cells. Consequently, it is expected that the substances are used as therapeutic agents for diseases that accompany sthesia of angiogenesis, for example, proliferation of solid cancers, diabetic retionpathy and psoriasis.

The angiogenesis inhibiting activity of the compound of the present invention can be measured by using inhibition of proliferation of human umbilical cord vein endothelial cells as an index as described below. That is, 0.1 ml of human umbilical cord vein endothelial cells (Cell Systems Corporation) adjusted to 2 × 10³ cells/well in MCDB131 medium (Chlorella Industry Co., Ltd.) containing 10 µg/l basic fibroblast cell growth factor and 10% fetal calf serum is seeded on a collagen-coated 96-well microplate (Sumitomo Bakelite Co., Ltd.) and at the same time a suitable amount of sample is added. The resultant is cultivated in an atmosphere containing 5% CO₂ at 37°C for 48 hours. Further, 1 µCi/well ³H-tymidine is added and cultivation is performed for 8 hours. Then, the cells are collected using a cell harvester and the radioactivity incorporated into cells is measured using a scintillation counter.

When the sample contains an active substance for inhibiting the growth of angioendothelial cells, the incorporation of radioactivity into the cells is suppressed. Further, an effective concentration range of the sample is obtained from the change in radioactivity in the cells depending on the concentration of the sample.

As shown in Table 3 below, the substances F-1941A to F-1941H of the present invention suppressed the growth of human funicular vein endothelial cells in low concentrations.

**Table 3**

| Substances | 50% growth inhibition concentration (mg/L) |
|---|---|
| F-1491A | 2.2 |
| F-1491B | 3.4 |
| F-1491C | 7.4 |
| F-1491D | 4.6 |
| F-1491E | 3.2 |
| F-1491F | 9.3 |
| F-1491G | 7.7 |
| F-1491H | 5.6 |

Further, as shown in Table 4 below, the substances F-1941A to F-1941H of the present invention showed substantially no toxicity to various cultivated cells.

**Table 4**

| Cells | 50% growth inhibition concentration (mg/L) | | | | | | | |
|---|---|---|---|---|---|---|---|---|
| | F-1491A | F-1491B | F-1491C | F-1491D | F-1491E | F-1491F | F-1491G | F-1491H |
| K562 | 39 | 36 | 52 | 80 | 68 | 72 | 95 | 61 |
| H226 | >100 | >100 | >100 | >100 | >100 | >100 | >100 | >100 |
| DLD-1 | >100 | >100 | >100 | >100 | >100 | >100 | >100 | >100 |
| HT1080 | >100 | >100 | >100 | >100 | >100 | >100 | >100 | >100 |

Note that the tests were performed as follows. Various kinds of human cancer cells (K562 (leukemia), H226 (lung cancer), DLD-1 (prostate cancer) and HT1080 (fibrosarcoma)) were suspended in RPMI1640 medium or DMEM medium containing 10% fetal calf serum and each adjusted to 5 × 10³ cells/well, and 0.1 ml of this was seeded on a 96-well microplate. At the same time, a suitable amount of each of the substances F-1491 (substances F-1491A to F-1491H) was added and the resultant was cultivated at 37°C for 72 hours in an atmosphere containing 5% CO₂. Further, 10 µl of 0.5% MTT (3-[4,5-dimethylthiazol-2-yl]2,5-biphenyl tetrazolium bromide) was added and the resultant was cultivated for 4 hours. After the cultivation, 0.1 ml of 10% SDS-0.01N hydrochloric acid was added and absorbance at 570 nm was measured to determine 50% growth inhibition concentration.

### BEST MODE FOR CARRYING OUT THE INVENTION

Hereinafter, the present invention will be described by examples. However, the present invention should not be considered to be limited by the following description.

### Example 1: Isolation of substances F-1491A and F-1491B

A loopful of *Aspergillus* sp. F-1491 (FERM BP-8288) strain on a slant medium (potato dextrose agar) was inoculated in a 500-ml Erlenmeyer flask containing 50 ml of a seed medium (2% potato starch, 1% glucose, 2% soybean powder ("Esusan Meat", manufactured by Ajinomoto), 0.1% potassium dihydrogen phosphate and 0.05% magnesium sulfate, without pH adjustment) and incubated on a rotary shaker at 25°C for two days to obtain a seed culture broth. Amedium consisting of 2% glycerol, 1% peptone (manufactured by Kyokuto Pharmaceutical Industrial Co., Ltd.), 0.5% yeast extract and 0.5% dried yeast (Ebios: manufactured by Asahi Breweries Chemicals Co., Ltd.) was used as a production medium. 60 ml each of the medium was charged in a 500-ml Erlenmeyer flask and sterilized. Then 1% each of the seed culture was inoculated. The flasks were incubated on a rotary shaker at 25°C for 4 days and 10 liters of the obtained culture broth was centrifuged to separate it into a culture filtrate and mycelium.

The obtained culture filtrate was passed through a 2-liter column packed with adsorbent resin Diaion HP-20 (manufactured by Mitsubishi Chemical Corporation), equilibrated with water. The HP-20 column on which the active ingredient adsorbed was washed with 5 liters of 50% aqueous methanol solution and then the active ingredient was eluted with 3 liters of methanol. The methanol was evaporated from the eluted solution under reduced pressure.

On the other hand, the mycelium was extracted with 3 liters of methanol and then the methanol was evaporated under reduced pressure. Those two were combined and extracted with 1 liter of ethyl acetate. The extract solution was concentrated under reduced pressure to obtain 7.5 g of a brown oily substance. The substance was dissolved in a small amount of chloroform and the solution was charged in a silica gel column (500 ml) filled with chloroform. The column was eluted with 1 liter of chloroform and then with 1 liter of a chloroform-methanol mixed solution (50:1). The fractions containing the substances F-1491A and F-1491B were collected and concentrated under reduced pressure to obtain 1.2 g of a yellow oily substance.

The substance was dissolved in a small amount of toluene and the solution was charged in a silica gel column (200 ml) filled with toluene. After having been washed with a toluene-acetone mixed solution (20:1), the column was developed with a toluene-acetone mixed solution (10:1). The fractions containing the substances F-1491A and F-1491B were collected and concentrated under reduced pressure to obtain 200 mg of a yellow powdery substance.

The substance was dissolved in a small amount of methanol and separated with a preparative high performance liquid chromatography. An Inertsil ODS-3 (manufactured by GL Science Co., Ltd.) of 20 mm I.D. x 250 mm was used as the separation column, and the column was eluted with an acetonitrile-water mixed solution (7:3) as a mobile phase at a flow rate of 7 ml/min to collect fractions containing the substances F-1491A and F-1491B. The respective fractions were concentrated under reduced pressure to obtain 15 mg of crude substance F-1491A and 13 mg of crude substance F-1491B. Further, the respective fractions were dissolved in small amounts of methanol and charged in 200-ml Sephadex LH-20 columns (manufactured by Pharmacia AB) and eluted with methanol. Active fractions were collected to obtain 10 mg of the substance F-1491A and 7 mg of the substance F-1491B.

### Example 2: Isolation of substances F-1491C to F-1491H

The fermentation was performed in the same manner as in Example 1. The culture broth filtrate (130 liters) was passed through 125-liter adsorbent resin Diaion HP-20 columns (manufactured by Mitsubishi Chemical Corporation) equilibrated with water. After the HP-20 column on which the active ingredient had been adsorbed was washed with 30 liters of 50% aqueous methanol solution, the active ingredient was eluted with 15 liters of methanol. The methanol was evaporated from the eluted solution under reduced pressure.

On the other hand, the mycelium was extracted with 15 liters of methanol and then the methanol was evaporated under reduced pressure. Those two were combined and extracted with 4 liters of ethyl acetate. The extract solution was concentrated under reduced pressure to obtain 80 g of a brown oily substance. The substance was dissolved in a small amount of chloroform and the solution was charged in a silica gel column (1,000 ml) filled with chloroform. After having been washed with 2 liters of chloroform, the column was eluted with 2 liters of a chloroform-methanol mixed solution (25:1) to collect fractions containing the substances F-1491C, D, E, F and G. Those were collected and concentrated under reduced pressure to obtain 12 g each of yellow oily substances. Further, the columns were eluted with 2 liters of a chloroform-methanol mixed solution (15:1) to collect fractions containing the substance F-1491H, which were then concentrated under reduced pressure to obtain 7 g of a yellow oily substance.

The respective substances were dissolved in small amounts of toluene and the respective solutions were charged in silica gel columns (400 ml) filled with toluene. The fractions containing the substances F-1491C, D, E, F and G were washed with a toluene-acetone mixed solution (20:1) and were developed with a toluene-acetone mixed solution (10:1). The fractions containing the substances F-1491C, D, E, F and G were collected and concentrated under reduced pressure to obtain 1.2 g of a yellow powdery substance. The fraction containing the substance F-1491H was washed with a toluene-acetone mixed solution (10:1) and then developed with a toluene-acetone mixed solution (3:1) to collect the fraction containing the substance H. Then, the fraction was concentrated under reduced pressure to obtain 0.8 g of a yellow powdery substance.

The substance was dissolved in a small amount of methanol and separated in a preparative high performance liquid chromatography. An Inertsil ODS-3 (manufactured by GL Science Co., Ltd.) of 20 mm I.D. x 250 mm was used as the separation column, and the column was eluted with an acetonitrile-water mixed solution (7:3) as a mobile phase at a flow rate of 7 ml/min to collect fractions containing the substances F-1491C, D, E, F and G, respectively. The respective fractions were concentrated under reduced pressure to obtain 10 mg of each of the substances F-1491C, D, E, F and G. Further, the fraction containing the substance F-1491H was eluted with an acetonitrile-water mixed solution (35:65) as a mobile phase at a flow rate of 7 ml/min to collect fractions containing the substance F-1491H. The fractions were concentrated under reduced pressure to obtain 15 mg of the substance F-1491H.

### INDSUTRIAL APPLICABILITY

Since the series of substances F1491 (F-1491A to F-1491H) have excellent angiogenesis inhibiting activity and low toxicity to cultivated cells, it is expected that the substances can be used as therapeutic agents for diseases that accompany sthesia of angiogenesis.

## Claims

1. Compounds represented by formula (I) 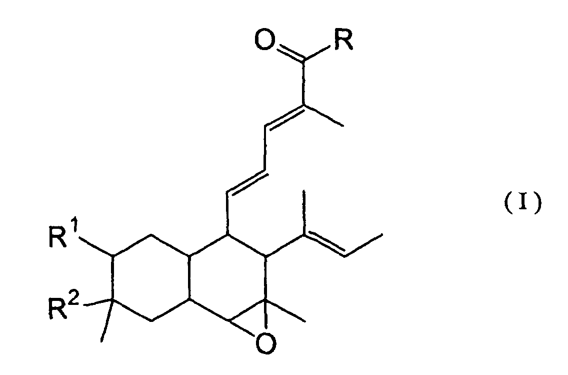 wherein R represents a methyl group or an ethyl group, R¹ represents a hydrogen atom, a chlorine atom, a hydroxyl group, or a methoxy group, R² represents a hydroxyl group, or R¹ and R² taken together form an epoxy ring structure.

2. The compound according to claim 1, wherein in formula (I), R represents an ethyl group, and R¹ and R² taken together form an epoxy ring structure.

3. The compound according to claim 1, wherein in formula (I), R represents a methyl group, and R¹ and R² taken together form an epoxy ring structure.

4. The compound according to claim 1, wherein in formula (I), R represents an ethyl group, R¹ represents a chlorine atom, and R² represents a hydroxyl group.

5. The compound according to claim 1, wherein in formula (I), R represents a methyl group, R¹ represents a chlorine atom, and R² represents a hydroxyl group.

6. The compound according to claim 1, wherein in formula (I), R represents an ethyl group, R¹ represents a hydrogen atom, and R² represents a hydroxyl group.

7. The compound according to claim 1, wherein in formula (I), R represents a methyl group, R¹ represents a hydrogen atom, and R² represents a hydroxyl group.

8. The compound according to claim 1, wherein in formula (I), R represents a methyl group, R¹ represents a methoxy group, and R² represents a hydroxyl group.

9. The compound according to claim 1, wherein in formula (I), R represents a methyl group, and R¹ and R² represent a hydroxyl group each.

10. A process for producing the compounds according to claim 1,
**characterized by**:
culturing a microorganism having the capability of producing the compounds according to claim 1; and
isolating the compounds according to claim 1 from the culture broth of the microorganism.

11. An Aspergillus sp. F-1491 (FERM BP-8288) strain having the capability of producing the compounds according to claim 1.

12. An angiogenesis inhibitory agent containing the compounds according to claim 1 as an active ingredient.
